# EUROPEAN PATENT APPLICATION

(11) **EP 1 782 855 A1**
(43) Date of publication of application: **09.05.2007**
(21) Application number: 06022585.1
(22) Date of filing: 30.10.2006
(51) Int. Cl.: A61M 27/00

(54) **Apparatus for delayed pericardial drainage**

(30) Priority: 03.11.2005 US 738679 P
(71) Applicant: MEDICAL INSTRUMENTS S.p.A., 40068 San Lazzaro Di Savena BO (IT)
(72) Inventor: De Paulis, Ruggero, 00151 Roma (IT)
(74) Representative: Alagem Modiano, Lara S.

(57) **Abstract**

Device for placement in the thoracic cavity of a patient. The device is a cannula (10), tube or catheter for pericardial drainage and serves as a conduit for drainage of excessive fluid in the pericardial cavity to a receptacle outside the body. The device is placed in the pericardial cavity at the time of surgery and is used for drainage of fluid collection that is formed at a later stage when all other drainage tubes have been already removed or are clogged by organic material. The device incorporates a system for preventing that any clog or material might close all fenestrations that are present in the tube at a plurality of sites.

## Description

The present invention related to a catheter, tube or cannula for delayed drainage of blood, fluid or contaminants from the pericardial cavity.

The field of this invention is cardiac surgery, thoracic surgery, trauma and critical care.

Chest drainage tubes or pericardial drainage tubes are used following cardiac, thoracic surgery, chest trauma or for specific medical conditions. The purpose of a chest or pericardial drainage is to remove build-up of excessive body fluid (blood or serum) from the pericardial and mediastinal cavity. An excess of fluid into the pericardial cavity might result in compression of the cardiac chambers with consequent impairment of the pump function causing a condition called cardiac tamponade.

Current pericardial or chest drainage cannulae, also called pericardial or chest tubes, drainage catheters or drainage cannulae are flexible polymer tubes, placed into the chest cavity and extending outside the patient.

Chest or pericardial drainage catheters are usually positioned at the end of a cardiac surgical procedure or after a chest surgery involving the pericardial cavity, before closing the surgical incision of the chest. In the hours following the surgical procedure, blood, fluid or other contaminants that build up inside the pericardial cavity are forcefully removed by use of external vacuum or pumping system. The chest or pericardial tube drainages are usually left in situ for a period that might vary from 24 to 48 hours after the end of surgery or until the amount of fluid that is being drained is drastically reduced. The tube drainages are then removed.

However, in some cases the fluid build up might start or continues after pericardial or chest tubes have been removed, In other cases the pericardial or chest tubes might have clogged early after surgery causing an incomplete fluid drainage. In other cases late lysis of clotted blood might be the cause of fluid production. In all these cases it become necessary to proceed to evacuate the fluid from the pericardial cavity either by surgically re-exploring the chest or by a percutaneous insertion of a catheter into the pericardial cavity. This latter procedure is usually accomplished by X-ray or ultrasound guidance and involves a risk of iatrogenic trauma to the patient.

The insertion of an improved pericardial drainage tube at the time of surgery would allow the medical team to drain from the pericardial cavity any fluid that is produced at a later stage when all other chest tubes have been already removed. In this way any additional invasive procedure is prevented and the postoperative care of the patient is simplified.

The invention relates to a catheter, tube or cannula for delayed drainage of blood, fluid or contaminants from the pericardial cavity.

The cannula of the present invention is a standard polymeric tube (with or without a metal spiral winding to prevent kinking) which is fenestrated near the distal tip at a plurality of sites. The cannula includes an internal shaft of similar polymeric material that fills the tube cavity. This internal shaft has a plurality of small appendages of the same polymeric material corresponding in number and position to the fenestrations that are present along the distal tip of the external tube. In this way the internal shaft completely fills the external tube and with its small appendages completely occludes the tube fenestrations.

Because of the presence of the internal shaft and its appendages, clotted blood, fibrin or other organic material cannot occlude the small fenestrations that are present near the tip of the cannula even if the cannula remains inside the pericardial cavity for an extended period of time. When the presence of fluid is detected inside the pericardial cavity the internal shaft is removed, thereby opening all fenestrations present along the cannula. The pericardial cannula is then connected to an external vacuum or pumping system allowing the fluid to be actively drained outside.

The cannula of the present invention along with its internal shaft is positioned inside the pericardial cavity at the end of surgery along with other standard chest drains. Placement is accomplished by routing the cannula from the pericardial cavity, through the patient skin, and extending outside the body. The cannula is fixed to the patient skin and left in situ throughout all the postoperative period. The cannula of the present invention is to be left in situ after all standard chest tubes have been removed according to the standard protocols. Should a delayed pericardial effusion arise (most frequently for those patients who need to be on anticoagulant therapy, or those with a strong inflammatory response), simply removal of the internal shaft of the cannula will allow an immediate drainage of excess of fluid without the need for chest re-exploration or repositioning of other drainage catheters.
Figure 1 illustrates the external tubing with its multiple fenestrations near the tip;
Figure 1a is a sectional view of the external tubing;
Figure 2 illustrates the internal shaft of the cannula with its multiple appendages corresponding to the multiple fenestrations of the cannula;
Figure 2a is a sectional view of the internal shaft;
Figure 3 illustrates the cannula with the shaft inserted therein;
Figure 3a is a sectional view of the cannula and the shaft inserted therein.

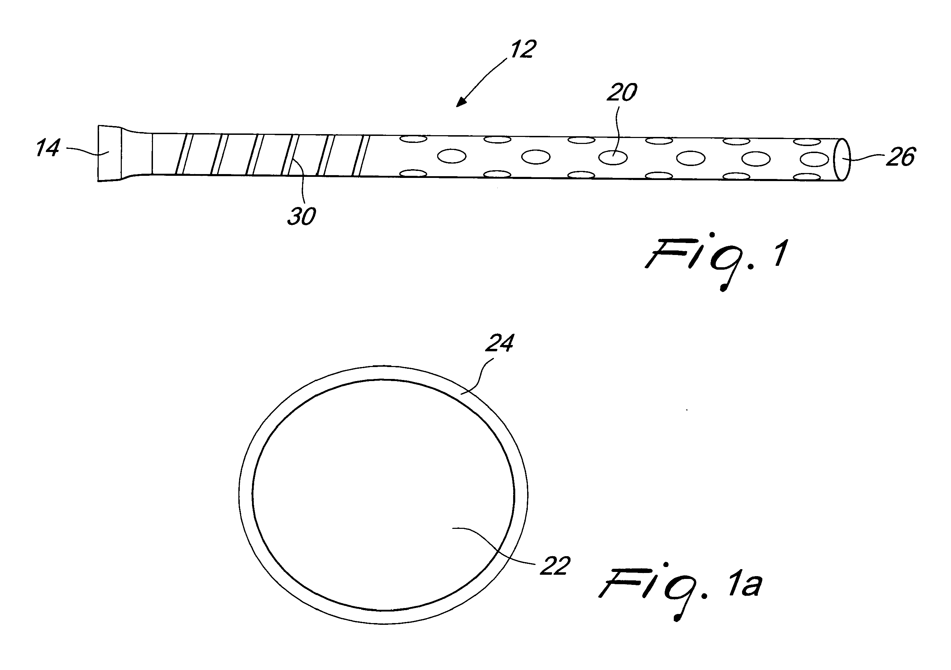

Figure 1 illustrates a tubing 12 that, along with an internal shaft, forms a cannula 10 of the present invention. The tubing 12 has a proximal end 14 to be connected with an external vacuum or pumping system and a distal end with a plurality of drainage holes 20 and an open ending 26. The tubing 12 preferably comprises a stiffening wire 30.

Figure 1a illustrates the cross sectional view of the tubing 12 that comprises a drainage lumen 22 and a wall 24. The tubing material may be selected from any polymer such as, but not limited to, polyvinyl chloride, polyurethane, polyethylene and the like.

The tube 12 is, preferably, transparent or semitransparent. At least a portion of the tubing 12 is preferably stiffened with a helical winding of material such as stainless steel, nitinol and the like. The stiffening 30 could also be provided using corrugations in the tubing 12 or by addition of a strong polymer such as glass-filled polycarbonate instead of the helical winding. The stiffening member 30 serves the purpose of preventing collapse of the tubing 12 when vacuum is applied to the drainage lumen 22. The stiffening member 30 also serves to prevent kinking when the tubing 12 is bent around a tight radius.

The distal end of the tubing 12 comprises a plurality of drainage holes 20 and an open ending 26. The drainage holes 20 connect the exterior of the tubing 12 with a drainage lumen 22. The holes 20 are of sufficient size and quantity to allow the passage of fluid, thrombus and debris that might need to be removed from the pericardial cavity or mediastinum. The plurality of drainage holes 20 and the drainage lumen 22 may further be coated with an anti-thrombogenic coating of material such as, but not limited to, heparin.

The proximal end 14 of the tubing 12 is capable of being connected to gravity-fed or vacuum-fed standard drainage systems. Standard drainage systems generally comprise a connector, a length of tubing and a reservoir. A vacuum system may be connected to the reservoir.

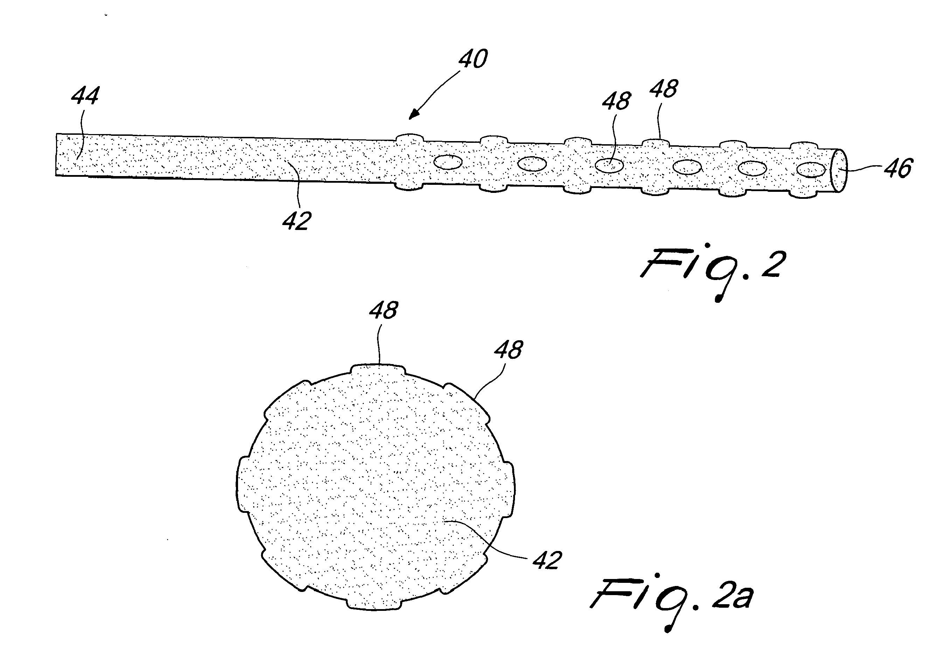

Figure 2 illustrates a shaft 40 that is positioned inside the drainage lumen 22 of tubing 12 to form the cannula 10 of the present invention. The shaft has a body 42, a proximal end 44 and a rounded distal end 46. It also comprises in the distal end a plurality of appendages 48 made of the same material, that correspond exactly in position and number to the plurality of drainage holes present along the distal end of the tubing 12. The shaft and its appendages are typically made of polymer preferably of the same type as that of the external tubing 12. In this way both the external tubing 12 and the internal shaft 40 are entirely malleable.

Figure 2a is a sectional view of the distal end of the internal shaft 40 with its appendages 48.

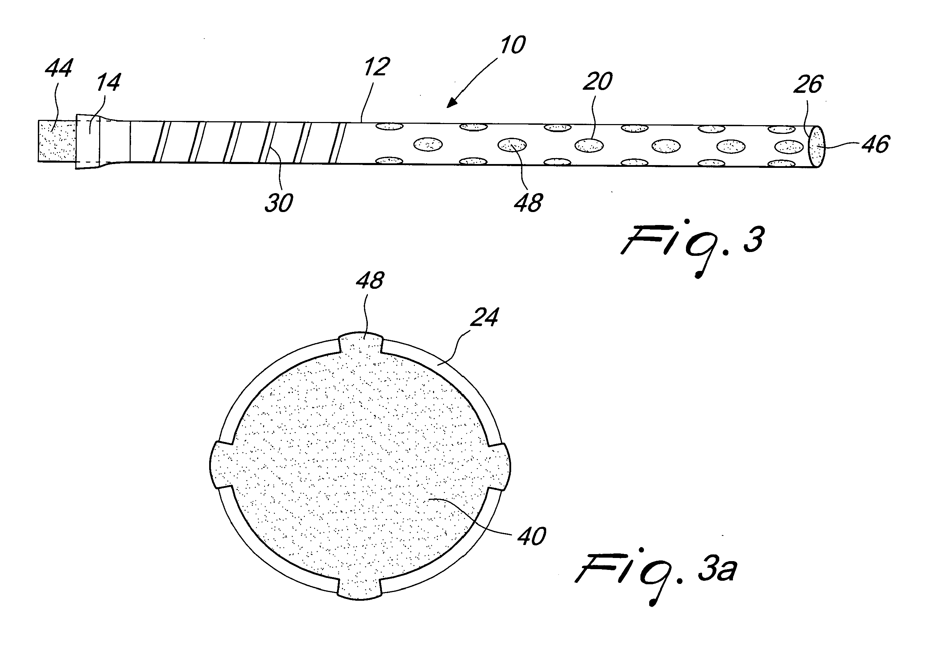

Figures 3 and 3a are respectively a long sectional view and a short sectional view of the cannula 10 of the present invention. The cannula comprises the external tubing 12 and the internal shaft 40. Once the internal shaft 40 is inserted inside the external tubing 12, all the appendages 48 of the shaft 40 totally fill the plurality of side holes 20 present on the distal end of the tubing 12. Similarly, the distal rounded end 46 of the shaft 40 occludes the open end 26 of the tubing 12. The external surface of the cannula 10 will then result smooth and uniform and fluid and debris cannot enter the lumen 22 of tubing 12 through the plurality of holes 20. The proximal end 44 of the shaft is slightly longer than the external tubing 12; in this way, when necessary, the internal shaft 40 can be pulled out thereby opening the plurality of side holes that are present in the tubing 12 allowing fluid and debris to enter the lumen 22 and be drained outside.

Operation of the cannula is as follows.

The cannula 10 of the present invention which comprises the external tubing 12 and its internal shaft 40 is positioned inside the pericardial cavity at the end of a standard cardiac or thoracic surgical procedure. Placement of the cannula is accomplished by positioning its distal end preferably at the bottom of the pericardial cavity and by routing the cannula from the pericardial cavity, through the patient skin, outside the body. The cannula is fixed to the patient skin using standard surgical suturing and left in situ troughout the whole postoperative period (usually 6-8 days). At the same time standard postoperative chest drainage tubings are positioned inside the chest, and connected with standard suction devices to allow any excess of blood and fluid to be drained outside the body. The continuous monitoring of blood loss in the postoperative period is extremely important since any unexpected bleeding can be immediately noticed and the most appropriate treatment (usually chest re-exploring) promptly carried out. When the amount of blood and fluid slowly decreases in time and virtually stops the chest tubes are removed. This is usually accomplished between 24 or 48 hours after surgery. Nonetheless, in some cases, either because of an excessive inflammatory response or because of the starting of an anticoagulant therapy or because of a lysis of blood clots among other causes, a certain amount of fluid slowly builds up inside the pericardial cavity. In most cases the amount of fluid is minimal and it does not cause any clinical problem. However, in a certain number of cases the presence of blood or serum inside the pericardial cavity can be the cause of fever, delayed reduction of the systemic inflammatory state, induction or maintenance of atrial fibrillation among other clinical or sub-clinical signs. In some more rare cases, the increase of fluid inside the pericardial cavity can reach a level where it compresses the cardiac cavity causing a number of clinical conditions that may vary from a small reduction in stroke volume up to a true cardiac tamponade. In all these cases the presence of the cannula 10 of the present invention allows the drainage of fluid outside without the need of any invasive procedure like repositioning of pericardial catheter or chest re-exploring. In fact, it is sufficient to pull the shaft of the present invention out of the external tubing 12 in order to open the plurality of drainage holes 20 that are present along the distal end of the cannula and allow any excess of blood or fluid to enter the lumen 22 and be drained outside the body. To improve the drainage the proximal end 14 of the cannula can be connected to a standard vacuum system.

The shaft 40 of the present invention with all its appendages 48 is in fact a system for preventing that any clot, clog or material might close all fenestrations that are present in the distal end of the cannula during the postoperative period.

The disclosures in US Provisional No. 60/738,679 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An apparatus adapted for drainage of fluid, air and contaminants from a thoracic cavity comprising:
- an axially elongated tubing with a proximal and distal end;
- a drainage lumen within said tube and extending substantially along the length of said tube;
- a plurality of distal openings into the drainage lumen;
- an internal shaft with a proximal and distal end;
- a plurality of appendages at the distal end of said shaft corresponding in number and position to the distal openings of the drainage tube.

2. A method of draining fluid, air and contaminants from a thoracic cavity comprising the steps of:
- providing a cannula with an internal shaft;
- positioning the cannula with its internal shaft inside a pericardial cavity and routing it through the skin, extending outside the body of a patient;
- pulling the shaft out of the cannula and opening a plurality of distal openings allowing fluid, air or contaminants to enter a drainage lumen and be drained outside.
